# EUROPEAN PATENT SPECIFICATION

(11) **EP 0 335 954 B1**
(45) Date of publication and mention of the grant of the patent: **10.05.1995**
(21) Application number: 88909419.9
(22) Date of filing: 29.09.1988
(51) Int. Cl.: G01N 21/78, G01N 33/52, G01N 33/72

(54) **METHOD FOR FORMING A STABLE REAGENT FOR DETERMINING BILIRUBIN IN SERUM AND REAGENT OBTAINABLE BY SAID METHOD**
METHODE ZUR HERSTELLUNG EINES STABILEN REAGENS ZUR BESTIMMUNG VON BILIRUBIN IN SERUM UND NACH DIESER METHODE ERHÄLTLICHES REAGENS
METHODE POUR LA PRODUCTION D'UN REACTIF STABLE DE DETERMINATION DE BILIRUBINE DANS UN SERUM ET REACTIF CONVENANT A LA MISE EN OEUVRE DE CETTE METHODE

(30) Priority: 29.09.1987 US 101776
(43) Date of publication of application: 11.10.1989
(73) Proprietor: Modrovich, Ivan E., Camarillo CA 93010 (US)
(72) Inventor: Modrovich, Ivan E., Camarillo CA 93010 (US)
(74) Representative: Grünecker, Kinkeldey, Stockmair & Schwanhäusser Anwaltssozietät
(86) International application number: US8803365
(87) International publication number: WO8903030

(56) References cited:
- EP-A- 0 119 861
- JP-A- 6 239 773
- JP-A-58 140 642
- US-A- 3 585 004
- US-A- 3 989 462
- US-A- 4 119 401
- US-A- 4 311 483
- US-A- 4 612 290
- PATENT ABSTRACTS OF JAPAN, vol. 7, no. 256 (P-236)[1401], 15th November 1983;& JP-A-58 140 642
- CLINICAL CHEMISTRY, vol. 32, no. 7, July 1986, pages 1389-1393; K.L.J. VINK etal.: "Use of the caffeine reagent in direct spectrophotometry of bilirubin"
- R.J. HENRY et al, "Clinical Chemistry, Principles and Technics", Second Edition, published 1974, by Harper & Row (New York), see pages 1045-1050, especially page 1047, lines 28-43

## Description

The present invention relates to a method for forming a time stable liquid assay composition for determination of bilirubin in biological fluids and a composition obtainable by said method.

### Background of the Invention

Bilirubin is a reddish yellow substance found in biological substance such as blood serum, and has the empirical formula: C₃₃H₃₆N₄O₆

Its presence in serum at too high a level indicates jaundice and its measurement is used as a liver function test. There are assays known for determining the presence of bilirubin in biological substances. JP-A-6239773 discloses a solid assay system, comprising a water impermeable carrier laminated with a water absorbing layer and a porous reagent layer, the latter containing a diazonium salt. The elements contain an ionic surfactant.

Further, there are liquid assay compositions known.

One standard assay used for the quantitive determination of bilirubin is the Jendrassik-Grof method which uses as the assay composition an aqueous solution of caffeine, a benzoate salt, an acetate salt and diazonium salt of sulfanilic acid formed by reacting sulfanilic acid and sodium nitrite in diluted hydrochloric acid. (See e.g. JP-A-58140642)

Bilirubin present in the serum reacts with the diazotized sulfanilic acid to form a chromophore in which the caffeine and benzoate and acetate salts cooperate to serve as an activator and speed the reaction.

The problem with the solution used is its stability. It is initially supplied as a three component system. One contains caffeine, the benzoate salt and the acetate salt. The second contains sulfanilic acid and hydrochloric acid. The third reagent contains sodium nitrite and is the least stable of the three. When the three components are combined, stability is less than 8 hours.

It would be desirable, therefore, to have a stable single reagent system which enables the measurement of bilirubin. Such a system would be lower in cost as three different components need not be packaged. It would also reduce the chance of human error which can occur in combining three components to make an assay reagent. This is because better quality control exist when all components can be added to form a single reagent in quite precise amounts. Such a reagent of adequate shelf life has not heretofore been proposed in the art ready to use in liquid form.

### Summary of the Invention

The present invention provides a single reagent system of an excellent shelf stability of at least 18 months at 2 to 8°C and is highly responsive to the quantitative measurement of bilirubin concentration in biological fluids such as blood serum at 540 nanometers (nm).

The composition obtainable according to the method of claim 1 comprises water and the following ingredients: a complexing agent present in concentrations sufficient to complex heavy metals, from about 1 g/l to solution saturation of a water soluble organic sulfate, from 0 to 100 g/l caffeine and from about 0.05 to about 15 mM of at least one diazonium salt, said composition having a pH from about 1.0 to about 6.0 and being stable at least 18 months at 2°C to 8°C. The water soluble organic sulfate may be a long chain aliphatic sulfate, an aromatic sulfate or mixtures thereof. A preferred diazonium salt is 3,5-dichlorophenol diazonium tetrafluoro-borate at a concentration of about 0.15 mM, the preferred concentration of caffeine is from about 2 to about 100 g/l. Normally, EDTA concentration is about 2 g/l. Solution pH is from about 1.0 to about 6,0 preferably about 3.5 to about 5.5, and most preferably about 4.4.

The composition is prepared by first adding the complexing agent (EDTA) to water followed by dissolving the organic sulfate then dissolving the caffeine and finally dissolving the diazonium salt.

The resulting mixture is stable for at least 18 months at 2 to 8°C which is a time sufficient to enable manufacture, worldwide shipment, warehousing and storage until use.

### Detailed Description

There is provided in accordance with the present invention a single reagent composition of excellent shelf life which enables the quantitive assay of bilirubin concentration in blood serum. The composition is an aqueous solution of at least one diazonium salt, preferably 3,5 dichlorophenol diazonium tetrafluoroborate, present in a concentration from about 0.05 to about 15 millimoles per liter (mM), preferably more, about 0.05 to about 1.5 mM, more preferably about 0.15 mM; 1 g/l to solution saturation of a water soluble alkali salt of an organic sulfate where the organic moiety is a long chain aliphatic group, an aromatic group and mixtures thereof. The lithium salts are presently preferred and preferred organic sulfate is the lithium salt of dodecyl sulfate present in a preferred concentration of about 10 g/l. There is also present 0 to about 100 g/l preferably from about 2 to about 100 g/l, more preferably about 10 g/l caffeine and EDTA ethylenediaminetetraacetic acid (EDTA) in sodium salt form present in a concentration sufficient to complex with any heavy metal ion which are present. The current preferred concentration of EDTA is about 2 g/l.

The solution has a pH from about 1 to about 6, preferably about 4 to about 5, and most preferably about 4.4.

The composition is prepared by first adding EDTA to distilled water to complex any heavy metals which are present, the water soluble organic sulfates are added and dissolved, followed by addition of and dissolving of caffeine. The diazonium salt is added last. The solution as prepared is stable at temperatures of about 2 to about 8°C (refrigeration conditions) for at least 18 months.

In the system, the diazonium salt diazotizes with bilirubin forming a chromogen which is red in color. The water soluble organic sulfate acts to catalyze the diazo reaction, make the complex absorbance more intense and stabilizes the diazonium salt against degradation. Caffeine accelerates the formation of the complex and intensifies the color to make the absorbance higher.

In use, approximately one milliliter of the preferred composition and about 50 microliters of the serum are combined. Any bilirubin present is diazotized to form a red chromogen whose intensity is measured at 540 nanometers to eliminate interference attributed to hemaglobin, lipemia and the like which obscure the presence of bilirubin if absorbance is measured at 515 nanometers where maximum absorbance occurs. Making the determination at 540 nanometers, the interference is minimized and the bilirubin present reacts to provide the intense color enabling measurement.

The assay system of this invention is suited to the measurement of total bilirubin including unconjugated and/or conjugated fractions.

## Claims

1. A method for forming the timestable liquid assay composition suitable for determination of total bilirubin in biological fluids at 540 nanometers which comprises forming a solution by dissolving at least one complexing agent in water in an amount sufficient to complex heavy metal ions, followed by dissolving in the solution of water and complexing agent at least one water soluble organic sulfate to a concentration of from about 1 g/l to solution saturation, caffeine to a concentration of from 0 to about 100 g/l and then adding to the resultant solution at least one diazonium salt in a concentration of about 0.05 to about 15 mM of solution, to form a composition having a pH of about 1.0 to about 6.0 and being stable at least 18 months at 2°C to 8°C.

2. The method as claimed in claim 1 in which the at least one complexing agent is ethylenediaminetetraacetic acid present in a concentration of 2 g/l.

3. The method as claimed in claim 1 or 2 in which the at least one diazonium salt is 3,5 chlorophenol diazonium tetrafluoroborate.

4. The method as claimed in any of claims 1 to 3 in which at least one water soluble organic sulfate is selected from the group consisting of long chain aliphatic sulfates, aromatic sulfates and mixtures thereof.

5. The method as claimed in any of claims 1 to 4 in which at least one water soluble organic sulfate is a lithium salt of dodecyl sulfate.

6. The method as claimed in any of claims 1 to 5 in which the at least one diazonium salt is provided to a concentration of about 0.15 mM.

7. The method as claimed in any of claims 1 to 6 in which the formed composition has a pH of from about 3.5 to 5.5.

8. A timestable liquid assay composition for determination of bilirubin in biological fluids obtainable according to the method of Claim 1 and which comprises water and the following ingredients: a complexing agent present in concentrations sufficient to complex heavy metals, from about 1 g/l to solution saturation of a water soluble organic sulfate, from 0 to about 100 g/l caffeine and from about 0.05 to about 15 mM of at least one diazonium salt, said composition having a pH from about 1.0 to about 6.0 and being stable at least 18 months at 2°C to 8°C.

9. The composition as claimed in claim 8 in which caffeine is present in an amount of from about 2 to about 100 g/l.

10. The composition as claimed in claim 8 or 9 in which the diazonium salt is 3,5 chlorophenol diazonium tetrafluoroborate.

11. The composition as claimed in any of claims 8 to 10 in which the diazonium salt concentration of the solution is about 0.15 mM.

12. The composition as claimed in any of claims 8 to 11 in which the water soluble organic sulfate is selected from a group consisting of long chain aliphatic sulfate, aromatic sulfate and mixtures thereof.

13. The composition as claimed in claim 12 in which the water soluble organic sulfate is an alkali metal salt of dodecyl sulfate.

14. The composition as claimed in any of claims 8 to 13 in which the sulfate salt concentration of the solution is about 0.15 mM.

15. The composition as claimed in any of claims to 8 to 14 in which the solution has a pH of about 3.5 to 5.5.

16. The composition as claimed in any of claims 8 to 15 which the complexing agent is ethylenediaminetetraacetic acid present in a concentration of about 2 g/l.

17. The composition according to any of claims 8 to 16 which comprises an aqueous solution containing about 2 g/l ethylenediaminetetraacetic acid, about 0.15 mM of 3,5 dichlorophenol diazonium tetrafluoroborate, about 10 g/l of lithium salt of dodecyl sulfate said solution having a pH of about 4.4 and being stable at 2°C to 8°C for at least 18 months.

18. The composition as claimed in any of claims 8 to 17 in which the formed composition has a pH of about 3.5 to 5.5.

## Patentansprüche

1. Verfahren zum Bilden einer langzeitstabilen flüssigen Testzusammensetzung, die für die Bestimmung von Gesamtbilirubin in biologischen Flüssigkeiten bei 540 nm geeignet ist, umfassend das Bilden einer Lösung durch Auflösen mindestens eines komplexbildenden Mittels in Wasser in einer Menge, die zur Komplexierung von Schwermetallionen ausreichend ist, gefolgt vom Auflösen mindestens eines wasserlöslichen organischen Sulfates in der Lösung aus Wasser und komplexbildendem Mittel in einer Konzentration von ungefähr 1 g/l bis zur Lösungssättigung und von Koffein in einer Konzentration von 0 bis ungefähr 100 g/l, und dann das Zufügen von mindestens einem Diazoniumsalz in einer Konzentration von ungefähr 0,05 bis ungefähr 15 mM der Lösung zu der resultierenden Lösung, um eine Zusammensetzung zu bilden, die einen pH von ungefähr 1,0 bis ungefähr 6,0 hat und bei 2°C bis 8°C mindestens 18 Monate stabil ist.

2. Verfahren nach Anspruch 1, in dem das mindestens eine komplexbildende Mittel Ethylendiamintetraessigsäure ist, die in einer Konzentration von 2 g/l vorhanden ist.

3. Verfahren nach Anspruch 1 oder 2, in dem das mindestens eine Diazoniumsalz 3,5-Chlorphenoldiazoniumtetrafluorborat ist.

4. Verfahren nach einem der Ansprüche 1 bis 3, in dem mindestens ein wasserlösliches organisches Sulfat aus der Gruppe ausgewählt ist, die aus langkettigen aliphatischen Sulfaten, aromatischen Sulfaten und Mischungen davon besteht.

5. Verfahren nach einem der Ansprüche 1 bis 4, in dem mindestens ein wasserlösliches organisches Sulfat ein Lithiumsalz von Dodecylsulfat ist.

6. Verfahren nach einem der Ansprüche 1 bis 5, in dem das mindestens eine Diazoniumsalz in einer Konzentration von ungefähr 0,15 mM bereitgestellt wird.

7. Verfahren nach einem der Ansprüche 1 bis 6, in dem die gebildete Zusammensetzung einen pH von ungefähr 3,5 bis 5,5 hat.

8. Langzeitstabile flüssige Testzusammensetzung für die Bestimmung von Bilirubin in biologischen Flüssigkeiten, erhältlich nach dem Verfahren gemäß Anspruch 1, die Wasser und die folgenden Bestandteile umfaßt: Ein komplexbildendes Mittel, das in Konzentrationen vorhanden ist, die ausreichend sind, Schwermetalle zu komplexieren, von ungefähr 1 g/l bis Lösungssättigung eines wasserlöslichen organischen Sulfates, von 0 bis ungefähr 100 g/l Koffein und von ungefähr 0,05 bis ungefähr 15 mM mindestens eines Diazoniumsalzes, wobei die Zusammensetzung einen pH von ungefähr 1,0 bis ungefähr 6,0 hat und bei 2°C bis 8°C mindestens 18 Monate stabil ist.

9. Zusammensetzung nach Anspruch 8, in der Koffein in einer Menge von ungefähr 2 bis ungefähr 100 g/l vorhanden ist.

10. Zusammensetzung nach Anspruch 8 oder 9, in der das Diazoniumsalz 3,5-Chlorphenoldiazoniumtetrafluorborat ist.

11. Zusammensetzung nach einem der Ansprüche 8 bis 10, in der die Diazoniumsalzkonzentration der Lösung ungefähr 0,15 mM beträgt.

12. Zusammensetzung nach einem der Ansprüche 8 bis 11, in der das wasserlösliche organische Sulfat aus einer Gruppe ausgewählt ist, die aus langkettigen aliphatischen Sulfaten, aromatischen Sulfaten und Mischungen davon besteht.

13. Zusammensetzung nach Anspruch 12, in der das wasserlösliche organische Sulfat ein Alkalimetallsalz von Dodecylsulfat ist.

14. Zusammensetzung nach einem der Ansprüche 8 bis 13, in der die Sulfatkonzentration der Lösung ungefähr 0,15 mM beträgt.

15. Zusammensetzung nach einem der Ansprüche 8 bis 14, in der die Lösung einen pH von ungefähr 3,5 bis 5,5 hat.

16. Zusammensetzung nach einem der Ansprüche 8 bis 15, in der das komplexbildende Mittel Ethylendiamintetraessigsäure in einer Konzentration von ungefähr 2 g/l ist.

17. Zusammensetzung nach einem der Ansprüche 8 bis 16, die eine wäßrige Lösung umfaßt, die ungefähr 2 g/l Ethylendiamintetraessigsäure enthält, ungefähr 0,15 mM 3,5-Dichlorphenoldiazoniumtetrafluorborat, ungefähr 10 g/l Lithiumsalz von Dodecylsulfat, wobei die Lösung einen pH von ungefähr 4,4 hat und bei 2 bis 8°C mindestens 18 Monate stabil ist.

18. Zusammensetzung nach einem der Ansprüche 8 bis 17, in der die gebildete Zusammensetzung einen pH von ungefähr 3,5 bis 5,5 hat.

## Revendications

1. Procédé de formation d'une composition d'essai liquide stable dans le temps convenant pour le dosage de la bilirubine totale dans des liquides biologiques à 540 nanomètres, qui comprend la formation d'une solution en dissolvant au moins un agent complexant dans de l'eau dans une quantité suffisante pour complexer les ions de métaux lourds, puis en dissolvant dans la solution d'eau et d'agent complexant au moins un sulfate organique soluble dans l'eau à une concentration d'environ 1 g/litre jusqu'à la saturation de la solution, de la caféine à une concentration de O à environ 100 g/litre, puis en ajoutant à la solution obtenue au moins un sel de diazonium à une concentration d'environ 0,05 à environ 15 mM, pour former une composition ayant un pH d'environ 1,0 à environ 6,0 et qui est stable pendant au moins 18 mois à 2 à 8 °C.

2. Procédé selon la revendication 1, dans lequel l'au moins un agent complexant est l'acide éthylènediamine tétracétique présent à une concentration de 2 g/litre.

3. Procédé selon les revendications 1 ou 2, dans lequel l'au moins un sel de diazonium est le tétrafluoroborate de 3,5-chlorophénol diazonium.

4. Procédé selon l'une quelconque des revendications 1 à 3, dans lequel l'au moins un sulfate organique soluble dans l'eau est choisi parmi des sulfates aliphatiques à longue châine, des sulfates aromatiques et des mélanges de ceux-ci.

5. Procédé selon l'une quelconque des revendications 1 à 4, dans lequel le sulfate organique soluble dans l'eau est le dodécylsulfate de lithium.

6. Procédé selon l'une quelconque des revendications 1 à 5, dans lequel le sel de diazonium est à une concentration d'environ 0,15 mM.

7. Procédé selon l'une quelconque des revendications 1 à 6, dans lequel la composition formée a un pH d'environ 3,5 à 5,5.

8. Composition d'essai liquide stable dans le temps pour le dosage de la bilirubine dans des fluides biologiques pouvant être obtenue conformément au procédé de la revendication 1 et qui comprend de l'eau et les ingrédients suivants : un agent complexant présent à une concentration suffisante pour complexer les métaux lourds, d'environ 1g/litre jusqu'à la saturation de la solution d'un sulfate organique soluble dans l'eau, de 0 à environ 100 g/litre de caféine et un ou plusieurs sels de diazonium à une concentration d'environ 0,05 à environ 15 mM, cette composition ayant un pH d'environ 1,0 à environ 6,0 et étant stable au moins 18 mois à 2 à 8 °C.

9. Composition selon la revendication 8, dans laquelle la caféine est présente dans une quantité d'environ 2 à environ 100 g/litre.

10. Composition selon les revendications 8 ou 9, dans laquelle le sel de diazonium est le tétrafluoroborate de 3,5-dichlorophénol diazonium.

11. Composition selon l'une quelconque des revendications 8 à 10, dans laquelle la concentration du sel de diazonium de la solution est d'environ 0,15 mM.

12. Composition selon l'une quelconque des revendications 8 à 11, dans laquelle le sulfate organique soluble dans l'eau est choisi parmi les sulfates aliphatiques à longue chaîne, les sulfates aromatiques et des mélanges de ceux-ci.

13. Composition selon la revendication 12, dans laquelle le sulfate organique soluble dans l'eau est un dodécylsulfate de métal alcalin.

14. Composition selon l'une quelconque des revendications 8 à 13, dans laquelle la concentration en sulfate de la solution est d'environ 0,15 mM.

15. Composition selon l'une quelconque des revendications 8 à 14, dans laquelle la solution a un pH d'environ 3,5 à 5,5.

16. Composition selon l'une quelconque des revendications 8 à 15, dans laquelle l'agent complexant est l'acide éthylènediamine tétracétique à une concentration d'environ 2 g/litre.

17. Composition selon l'une quelconque des revendications 8 à 16, qui comprend une solution aqueuse contenant environ 2 g/litre d'acide éthylènediamine tétracétique, du tétrafluoroborate de 3,5-dichlorophénol diazonium environ 15 mM, environ 10 g/litre de dodécylsulfate de lithium, cette solution ayant un pH d'environ 4,4 et étant stable à 2 à 8 °C pendant au moins 18 mois.

18. Composition selon l'une quelconque des revendications 8 à 17, dans laquelle la composition formée a un pH d'environ 3,5 à 5,5.
